Europäisches Patentamt

European Patent Office

Office européen des brevets

(19)

(11) Numéro de publication: **0 157 688**
**B1**

(12) **FASCICULE DE BREVET EUROPEEN**

(45) Date de publication du fascicule du brevet:
09.03.88

(21) Numéro de dépôt: **85400509.7**

(22) Date de dépôt: **15.03.85**

(51) Int. Cl.⁴: **A 61 B 6/08,** A 61 B 6/00

(54) **Installation de radiologie à filtre compensateur.**

(30) Priorité: **20.03.84 FR 8404287**

(43) Date de publication de la demande:
**09.10.85 Bulletin 85/41**

(45) Mention de la délivrance du brevet:
**09.03.88 Bulletin 88/10**

(84) Etats contractants désignés:
**DE NL**

(56) Documents cités:
**EP - A - 0 076 180**
**DE - A - 3 023 399**
**DE - B - 1 009 763**
**FR - A - 2 078 378**
**FR - A - 2 116 168**
**GB - A - 2 017 450**

**IEE TRANSACTIONS ON BIO-MEDICAL ENGINEERING,**
**volume BME-21, no. 3, mai 1974, pges 243-244, New**
**York, US; E.E. HOEFER et al.: "Computer-controlled**
**synthesis of tomograms by means of a TV storage**
**tube"**

(73) Titulaire: **THOMSON-CGR, 13, square Max-Hymans,**
**F-75015 Paris (FR)**

(72) Inventeur: **Klausz, Rémy, THOMSON-CSF SCPI 173, bld**
**Haussmann, F-75379 Paris Cedex 08 (FR)**

(74) Mandataire: **Chaverneff, Vladimir et al, THOMSON-CSF**
**SCPI 19, avenue de Messine, F-75008 Paris (FR)**

## Description

L'invention concerne une installation de radiologie à filtre(s) compensateur(s) et se rapporte plus particulièrement à de nouveaux moyens de positionnement d'un tel filtre.

On sait que, dans une installation de radiologie, les différences d'épaisseur et de densité de la partie du patient qui se trouve dans le champ de rayons X, sont parfois telles que la «modulation» du faisceau par le sujet excède la dynamique du détecteur. Il est connu d'interposer des structures de forme et d'épaisseur déterminées en fonction, notamment de la nature (densité moyenne) des tissus au-dessus desquels elles doivent être placées. Ces structures ou filtres compensateurs comportant des éléments à positionnement réglables, ont longtemps été réalisés en aluminium. Plus récemment, on a proposé des filtres en matière plastique transparente chargée au plomb, ce qui permet notamment de conserver le faisceau lumineux de positionnement (symbolisant le faisceau de rayons X) pendant la mise en place et le réglage des filtres. Néanmoins, l'efficacité de ces réglages dépend en grande partie de l'habileté de l'opérateur à moins de les réaliser en mode de fonctionnement radioscopique, ce qui présente d'autres inconvénients. En effet, d'une part le patient est soumis à une dose supplémentaire de rayonnement non directement utile au diagnostic mais d'autre part, pendant toute la durée de son service, l'opérateur a fréquemment les mains et les avant-bras plongés dans le faisceau de rayons X.

On connaît d'après les documents FR-A-2078378 et FR-A-2116168 des dispositifs compensateurs d'exposition pour appareil de radiographie, mais aucun de ces documents ne révèle de moyens simples permettant de régler rapidement ces dispositifs compensateurs.

L'invention vise au moins à permettre un positionnement plus précis et plus rapide du ou des filtres compensateurs. Elle a aussi pour objet de réduire autant que possible les doses de rayonnement reçu aussi bien par le patient que par l'opérateur.

Dans cet esprit, l'invention concerne principalement une installation de radiologie comprenant une source de rayons X, un récepteur de rayons X, une table porte-patient et au moins un filtre compensateur destiné à uniformiser l'exposition moyenne en variant l'absorption des rayons X par un positionnement ajustable dans un plan perpendiculaire à l'axe central du faisceau de rayons X, ledit filtre compensateur étant normalement placé dans un volume dans lequel s'inscrit le faisceau de rayons X, caractérisée en ce qu'elle comporte des moyens de projection optique, pour projeter sur la table porte-patient une image radiologique élaborée par ledit récepteur, et des moyens pour déplacer ledit filtre compensateur, au moins pendant une phase de réglage, dans le volume dans lequel s'inscrit ledit faisceau de ladite image radiologique projetée et en ce que ledit filtre compensateur présente des propriétés d'adsorption partielle à la fois dans le domaine des rayons X et dans le domaine de la lumière visible et en ce que en tout point du filtre, l'atténuation est sensiblement proportionnelle à l'absorption des rayons X.

Selon un mode de réalisation possible, l'installation comporte des moyens de déviation du faisceau de ladite image agencés pour qu'une partie du volume dans lequel s'inscrit ledit faisceau de ladite image soit sensiblement confondue avec une partie du volume dans lequel s'inscrit ledit faisceau de rayons X, le filtre étant situé dans cette partie de volume commune.

Dans la plupart des installations modernes de radiologie, on dispose d'une mémoire d'image interconnectée entre le détecteur (généralement un amplificateur de luminance) et le moyen de visualisation. Pour résoudre complètement le problème de l'irradiation fréquente de l'opérateur, on peut exploiter cette mémoire dans le cadre de l'invention.

Plus précisément, l'invention concerne aussi une installation du type mentionné ce-dessus comportant une mémoire d'image reliée audit récepteur pour mémoriser une série d'informations numériques représentatives d'une image radiologique, ladite mémoire étant du type à lecture cyclique et autonome, caractérisée en ce qu'un projecteur vidéo ayant son entrée de signal reliée à la sortie de ladite mémoire est disposée de façon que son axe de projection optique soit sensiblement perpendiculaire à l'axe du faisceau de rayons X et en ce qu'un miroir est interposé dans le volume dans lequel s'inscrit ledit faisceau de rayons X, à 45° par rapport audit axe de projection optique.

Ainsi, une émission pulsée de la source de rayons X, de durée relativement brève, suffit pour inscrire dans la mémoire les informations représentatives d'une image radiologique, laquelle se trouve projetée sur le corps du patient (ou sur un écran placé sur celui-ci) ce qui permet de régler la position du filtre compensateur en l'absence de rayonnement X.

Dans le cas où, par exemple l'image radiologique est projetée en monochrome sur le patient, un réglage correct du filtre est obtenu lorsque l'image projetée présente des contrastes relativement faibles.

L'invention sera mieux comprise et d'autres avantages de celle-ci apparaîtront mieux à la lumière de la description qui va suivre d'un mode de réalisation actuellement préféré d'une installation conforme à son principe, donnée à titre d'exemple et faite en référence au dessin annexé dans lequel:

– la figure 1 représente schématiquement une installation de radiologie incorporant les perfectionnements conformes à l'invention; et

– la figure 2 représente schématiquement une variante de l'installation.

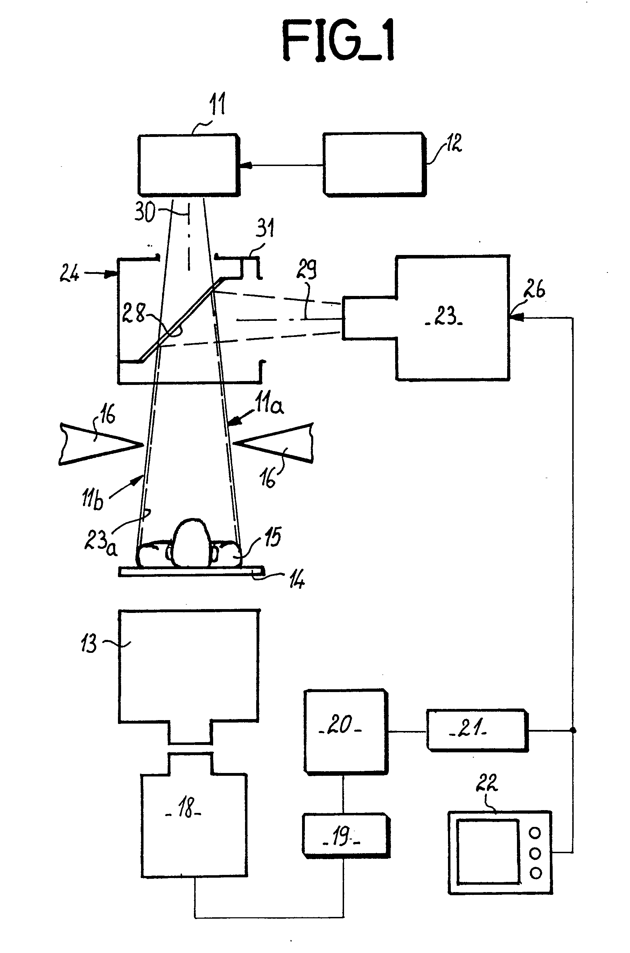

En se reportant à la figure 1, l'installation de radiologie représentée comporte une source de rayons X 11 alimentée par un générateur haute tension 12 et émettant un faisceau de rayons X 11a en direction d'un récepteur 13 constitué ici par un amplificateur de luminance. Une table porte-patient 14 est placée dans le faisceau de rayons X 11a, entre la source et le récepteur. Le sujet à examiner 15 est placé sur la table et, pour tenir compte des différences d'épaisseur et de densité moyenne des régions examinées situées dans le champ du faisceau 11a, on dispose un filtre compensateur 16, à positionnement réglable dans le faisceau 11a, entre la source et le sujet. La forme et l'épaisseur (variables) de ce filtre dépendent de la région du corps examinée. Une installation du type décrit comporte donc en tant qu'accessoires, un jeu de tels filtres avec des parties mobiles aux formes et dimensions différentes en fonction des principaux types d'examen radiologique.

L'installation comporte en outre une caméra de télévision 18 associée au récepteur 13 pour capter l'image radiologique formée dans ce dernier, un convertisseur Analogique-Numérique 19 recevant les signaux délivrés par la caméra, une mémoire d'image 20 connectée à la sortie du convertisseur 19, un convertisseur Numérique-Analogique 21 relié à la sortie de la mémoire et un récepteur de télévision 22 connecté à la sortie du convertisseur 21 et sur lequel s'affiche normalement l'image radiologique. Tous les sous-ensembles qui viennent d'être énoncés sont classiques et existent dans la plupart des installation de radiologie modernes. En particulier, la mémoire d'image 20 possède une capacité suffisante pour stocker sous forme numérique toutes les informations nécessaires à la reproduction d'une image reconstituée par le récepteur 13. Elle possède un mode de fonctionnement à lecture cyclique autonome, fonctionnant à un rythme suffisamment rapide pour maintenir une image visible sur l'écran du récepteur de télévision 22.

Pour la mise en œuvre de l'invention, les filtres 16 doivent présenter des propriétés d'absorption partielle à la fois dans le domaine des rayons X et dans le domaine de la lumière visible. On pourra utiliser pour cela des filtres en matière plastique chargée au plomb, absorbant partiellement la lumière, semblables à ceux qui sont fabriqués par exemple par la firme américaine «Nuclear Associates» mais comportant en outre un colorant assurant l'atténuation dans le domaine de la lumière visible. Les charges de plomb et de colorant seraient réglées de façon qu'en tous points du filtre, l'atténuation lumineuse soit sensiblement proportionnelle à l'atténuation des rayons X.

En outre, l'installation comporte des moyens de projection optique 23 de l'image radiologique élaborée par le récepteur 13 et des moyens de déviation 24 du faisceau de ladite image. Les moyens de déviation 24 sont agencés pour qu'une partie 23a du volume dans lequel s'inscrit le faisceau de l'image soit sensiblement confondue avec une partie 11b du volume dans lequel s'inscrit le faisceau de rayons X, le filtre compensateur 16 étant situé dans cette partie de volume commune.

Selon l'exemple, les moyens de projection optique 23 se composent essentiellement d'un projecteur vidéo dont l'entrée de signal 26 est reliée à la sortie du convertisseur 21, c'est-à-dire en aval de la mémoire 20 dans la chaîne de restitution de l'image radiologique décrite ci-dessus, tandis que les moyens de déviation 24 comportent essentiellement un miroir 28 interposé dans le volume dans lequel s'inscrit le faisceau de rayons X. Plus précisément, le projecteur 23 est disposé de façon que son axe de projection optique 29 soit perpendiculaire à l'axe 30 du faisceau de rayons X et le miroir 28 est monté dans un boitier 31 pour faire un angle de 45° par rapport à l'axe de projection optique 29. Le boitier 31 est interposé entre la sortie de la source 11 et l'emplacement des filtres 16. Si le miroir est fixé dans le boitier 24, il sera constitué d'une matière sensiblement radiotransparente. Ce type de miroir est classique, il existe déjà dans les collimateurs pour réfléchir le faisceau issu d'une ampoule à incandescence. Cependant, du fait que l'installation comporte une mémoire d'image 20, on peut aussi utiliser un miroir mobile (par exemple pivotant autour d'un axe latéral) permettant son dégagement du volume dans lequel s'inscrit le faisceau de rayons X, pendant le temps nécessaire à la formation de l'image et à sa mémorisation. La mise en œuvre de l'installation qui vient d'être décrite pour le réglage des filtres compensateurs est la suivante.

Le patient étant en position sur la table 14, on commande une émission pulsée de la source de rayons X 11, de durée brève juste nécessaire pour acquérir l'image radiologique et la mémoriser dans la mémoire 20. Pendant cette brève irradiation du patient, l'opérateur peut se tenir à distance. Puis, les informations lues cycliquement dans la mémoire 20 sont appliquées à l'entrée du projecteur vidéo 23 et l'image radiologique est projetée sur le corps même du patient après réflexion sur le miroir 28. L'opérateur dispose alors de tout le temps nécessaire pour manœuvrer les parties mobiles du filtre compensateur 16 de façon à atténuer les contrastes les plus importants de l'image projetée sur le patient. L'examen radiologique proprement dit peut alors commencer, en exploitant au mieux la dynamique de l'amplificateur de luminance 13.

D'autres moyens sont possibles pour projeter l'image radiologique sur le patient. Par exemple, le projecteur vidéo peut être remplacé par un agencement comprenant un générateur de faisceau laser, des moyens de balayage de ce faisceau et des moyens de modulation du faisceau reliés à la mémoire d'image 20. On peut aussi remplacer le projecteur vidéo par une simple lampe à incandescence de foyer relativement ponctuel et interposer entre le miroir 28 et le filtre compensateur 16 une lame plane définissant une matrice de cellules modulables en transmission, par exemple à cristaux liquides, les différentes cellules étant couplées à la mémoire image.

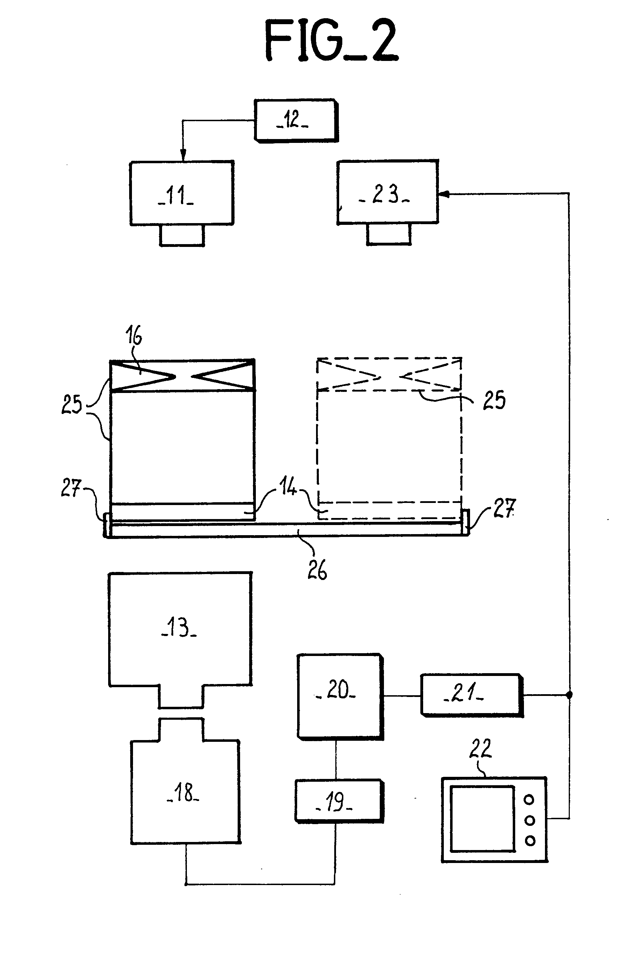

On peut aussi concevoir une installation dans laquelle le faisceau de rayons X et le faisceau de l'image ne comportent pas de partie commune pour le filtre. Le filtre est alors monté sur un support mobile entre deux positions prédéterminées, une position de réglage où il se trouve dans le faisceau de l'image et une position d'utilisation où il se trouve placé dans le faisceau de rayons X. La figure 2 illustre ce concept. Selon ce mode de réalisation où les éléments de structure analogues à ceux de la figure 1 portent les mêmes références numériques, le filtre 16 est monté dans un châssis-support 25 solidaire de la table porte-patient 14 et cette dernière est assujettie à se déplacer, latéralement le long de guides 26 entre deux positions prédéterminées par un système de butées 27 ou analogue. La première position représentée en trait plein sur la figure 2 est la position normale d'examen radiologique, la seconde position représentée en trait interrompu est telle que le projecteur vidéo 23 se substitue à la source de rayons X; c'est la position de réglage du filtre. Le mode d'utilisation de cette installation est donc des plus simples. Le patient est allongé sur la table 14 au-dessous du châssis-support du filtre 16 et la table est placée sous la source de rayons X 11. Une image radiologique est relevée dans ces conditions par mise en œuvre brève de la source 11. La table 14 est ensuite déplacée latéralement pour venir en position de réglage sous le projecteur vidéo 23. L'image radiologique est alors projetée pendant tout le temps nécessaire au réglage du filtre. La table 14 est ensuite ramenée en position d'examen sous la source de rayons X.

**Revendications**

1. Installation de radiologie comprenant une source de rayons X (11), un récepteur de rayons X (13), une table porte-patient (14) et au moins un filtre compensateur (16) destiné à uniformiser l'exposition moyenne en variant l'absorption des rayons X par un positionnement ajustable dans un plan perpendiculaire à l'axe central du faisceau de rayons X (30), ledit filtre compensateur étant normalement placé dans un volume (11a) dans lequel s'inscrit le faisceau de rayons X, caractérisée en ce qu'elle comporte des moyens de projection optique (23), pour projeter sur la table porte-patient (14) une image radiologique élaborée par ledit récepteur (13), et des moyens pour déplacer ledit filtre compensateur (16), au moins pendant une phase de réglage, dans le volume dans lequel s'inscrit ledit faisceau de ladite image radiologique projetée et en ce que ledit filtre compensateur (16) présente des propriétés d'absorption partielle à la fois dans le domaine des rayons X et dans le domaine de la lumière visible et en ce que en tout point du filtre, l'atténuation lumineuse est sensiblement proportionnelle à l'absorption des rayons X.

2. Installation selon la revendication 1, caractérisée en ce qu'elle comporte des moyens de déviation du faisceau de ladite image agencés pour qu'une partie (23a) du volume dans lequel s'inscrit ledit faisceau de ladite image soit sensiblement confondue avec une partie (11b) du volume dans lequel s'inscrit ledit faisceau de rayons X, ledit filtre compensateur étant situé dans cette partie de volume commune.

3. Installation selon la revendication 1, caractérisée en ce que ledit filtre est monté sur un support (25, 14) mobile entre deux positions prédéterminées, respectivement une position de réglage où ledit filtre se trouve dans le faisceau de ladite image et une position d'utilisation où ledit filtre se trouve dans le faisceau de rayons X.

4. Installation selon la revendication 3, caractérisée en ce que ledit support est solidaire d'une table porte-patient (14) mobile et que les deux positions prédéterminées précitées sont définies par un déplacement donné de ladite table.

5. Installation selon la revendication 3 ou 4, caractérisée en ce qu'elle comprend, de façon connue en soi, une mémoire d'image (20) reliée audit récepteur (18) pour mémoriser une série d'informations numériques représentatives d'une image radiologique, ladite mémoire étant du type à lecture cyclique et autonome et en ce que cette mémoire est reliée auxdits moyens de projection optique.

6. Installation selon la revendication 2 comprenant une mémoire d'image (20) reliée audit récepteur (18) pour mémoriser une série d'informations numériques représentatives d'une image radiologique, ladite mémoire étant du type à lecture cyclique et autonome, caractérisée en ce qu'un projecteur vidéo (23) ayant son entrée de signal (26) reliée à la sortie de ladite mémoire est disposé de façon que son axe de projection optique (29) soit sensiblement perpendiculaire à l'axe (30) du faisceau de rayons X et en ce qu'un miroir (28) est interposé dans le volume dans lequel s'inscrit ledit faisceau de rayons X, à 45° par rapport audit axe de projection optique.

7. Installation selon la revendication 6, caractérisée en ce que ledit miroir est fixe et sensiblement radio-transparent.

8. Installation selon la revendication 6, caractérisée en ce que ledit miroir est monté mobile pour pouvoir s'écarter du volume dans lequel s'inscrit ledit faisceau de rayons X.

9. Installation selon l'une des revendications précédentes, caractérisée en ce que ledit filtre compensateur est en matière plastique.

10. Installation selon la revendication 9, caractérisée en ce que ledit filtre compensateur est en matière plastique colorée, chargée au plomb.

**Patentansprüche**

1. Röntgeneinrichtung mit einer Röntgenstrahlenquelle (11), einem Röntgenstrahlenempfänger (13), einem Patiententrägertisch (14) und mindestens einem Kompensationsfilter (16), mit dem die mittlere Bestrahlung homogenisiert werden soll, indem die Absorption der Röntgenstrahlen durch eine wählbare Positionierung des Filters in einer Ebene senkrecht zur Mittelachse des Röntgenstrahls (30) variiert wird, wobei das Kompensationsfilter normalerweise in einem Volumen (11a) angeordnet ist, das vom Röntgenstrahl erreicht wird, dadurch gekennzeichnet, dass sie optische Projektionsmittel (23) zur Projizierung eines vom Empfänger (13) erarbeiteten Röntgenbilds auf den Patiententragetisch (14) und Mittel aufweist, um das Kompensationsfilter (16) zumindest während einer Einstellphase in dem Volumen zu verschieben, das der Röntgenstrahl des projizierten Röntgenbildes erreicht, und dass das Kompensationsfilter (16) sowohl im Röntgenbereich als auch im Bereich des sichtbaren Lichts Teilabsorptionseigenschaften besitzt, und dass in jedem Punkt des Filters die Lichtdämpfung im wesentlichen proportional zur Absorption der Röntgenstrahlen ist.

2. Einrichtung nach Anspruch 1, dadurch gekennzeichnet, dass sie Mittel zur Ablenkung des Strahls des Bildes aufweist, die so angeordnet sind, dass ein Teil (23a) des Volumens, der von dem Strahl des Bildes erreicht wird, im wesentlichen mit einem Teil (11b) des Volumens zusammenfällt, der vom Röntgenstrahl erreicht wird, wobei das Kompensationsfilter in diesem gemeinsamen Volumenteil liegt.

3. Einrichtung nach Anspruch 1, dadurch gekennzeichnet, dass das Filter auf einem Träger (25, 14) montiert ist, der zwischen zwei vorgegebenen Positionen beweglich ist, nämlich einer Einstellposition, in der das Filter sich im Strahl des Bildes befindet, und einer Verwendungsposition, in der das Filter sich im Röntgenstrahl befindet.

4. Einrichtung nach Anspruch 3, dadurch gekennzeichnet, dass der Träger mit einem beweglichen Patiententragtisch fest verbunden ist und dass die beiden vorgegebenen oben erwähnten Positionen durch eine gegebene Verschiebung des Tisches definiert sind.

5. Einrichtung nach Anspruch 3 oder 4, dadurch gekennzeichnet, dass sie in an sich bekannter Weise einen Bildspeicher (20) aufweist, der an den Empfänger (18) angeschlossen ist, um eine Reihe von digitalen Informationen zu speichern, die für ein Röntgenbild charakteristisch sind, wobei der Speicher zyklisch und autonom abgefragt wird, und dass dieser Speicher an die optischen Projektionsmittel angeschlossen ist.

6. Einrichtung nach Anspruch 2 mit einem Bildspeicher (20), der an den Empfänger (18) angeschlossen ist, um eine Reihe von für ein Röntgenbild repräsentativen digitalen Informationen zu speichern, wobei der Speicher zyklisch und autonom ausgelesen wird, dadurch gekennzeichnet, dass ein Videoprojektor (23), dessen Signaleingang (26) an den Ausgang des Speichers angeschlossen ist, so angeordnet ist, dass seine optische Projektionsachse (29) im wesentlichen senkrecht zur Achse (30) des Röntgenstrahls verläuft, und dass ein Spiegel (28) in das vom Röntgenstrahl bestrahlte Volumen mit einer Neigung von 45° bezüglich der optischen Projektionsachse eingefügt ist.

7. Einrichtung nach Anspruch 6, dadurch gekennzeichnet, dass der Spiegel ortsfest und im wesentlichen röntgenstrahlendurchlässig ist.

8. Einrichtung nach Anspruch 6, dadurch gekennzeichnet, dass der Spiegel beweglich montiert ist, um aus dem Volumen, das vom Röntgenstrahl erreicht wird, entfernt werden zu können.

9. Einrichtung nach einem der vorhergehenden Ansprüche, dadurch gekennzeichnet, dass das Kompensationsfilter aus Kunststoff ist.

10. Einrichtung nach Anspruch 9, dadurch gekennzeichnet, dass das Kompensationsfilter aus koloriertem Kunststoff ist, der Blei enthält.

**Claims**

1. An X-ray installation comprising an X-ray source (11), an X-ray receiver (13), a table (14) for bearing a patient and at least one compensation filter (16) intended to render uniform the mean exposition by varying the X-ray absorption by means of an adjustable positioning of the filter in a plane perpendicular to the central axis of the X-ray beam (30), said compensation filter being normally placed in a volume (11a) in which the X-ray beam is inscribed, characterized in that it comprises optical projection means (23) intended to project onto the table bearing the patient (14) a radiological picture produced by said receiver (13), and means for displacing said compensation filter (16), at least during an adjustment period, in the volume in which said beam of said projected radiological picture is inscribed, and that said compensation filter (16) presents partial absorption properties as well in the X-ray range as in the range of the visible light, and that the light attenuation is substantially proportional to the X-ray absorption in any point of the filter.

2. An installation according to claim 1, characterized in that it comprises means for deviating the beam of said picture, arranged such that a portion (23a) of the volume in which said beam of said picture is inscribed substantially coincides with a portion (11b) of the volume in which said X-ray beam is inscribed, said compensation filter being situated in this common volume portion.

3. An installation according to claim 1, characterized in that said filter is mounted on a support (25, 14) which can be moved between two predetermined positions, namely an adjustment position in which the filter is located in the beam of said picture, and a use position in which the filter is located in the X-ray beam.

4. An installation according to claim 3, characterized in that said support is fixed to a movable table (14) bearing the patient and that said two predetermined positions are defined by a given displacement of said table.

5. An installation according to claim 3 or 4, characterized in that it comprises, known per se, a picture memory (20) connected to said receiver (18) for storing a series of digital informations representing a radiological picture, said memory being of the cyclic and autonomous read-out type, and that said memory is connected to said optical projection means.

6. An installation according to claim 2, comprising a picture memory (20) connected to said receiver (18) for storing a series of digital informations representing a radiological picture, said memory being of the cyclic and autonomous read-out type, characterized in that a video projector (23), the signal input (26) of which is connected to the output of said memory, is disposed such that its optical projection axis (29) is substantially perpendicular to the axis (30) of the X-ray beam, and that a mirror (28) is inserted into the volume in which said X-ray beam is inscribed, at an angle of 45° with respect to the optical projection axis.

7. An installation according to claim 6, characterized in that said mirror is stationary and substantially radio-transparent.

8. An installation according to claim 6, characterized in that said mirror is mounted in a movable manner such that it can be withdrawn from the volume in which said X-ray beam is inscribed.

9. An installation according to one of the preceding claims, characterized in that said compensation filter is made of synthetic material.

10. An installation according to claim 9, characterized in that said compensation filter is made of coloured synthetic material, charged with lead.

0157688

# FIG_1

7

FIG_2

0157688

9